# EUROPEAN PATENT SPECIFICATION

(11) **EP 3 935 387 B1**
(45) Date of publication and mention of the grant of the patent: **18.06.2025**
(21) Application number: 20708017.7
(22) Date of filing: 05.03.2020
(51) Int. Cl.: G01N 33/52, G01N 1/30, G02B 21/34

(54) **STAINING AGENT SOLUTIONS FOR USE IN FLUORESCENCE MICROSCOPY**
FÄRBEMITTEL-LÖSUNGEN ZUR VERWENDUNG IN DER FLUORESZENZMIKROSKOPIE
SOLUTIONS D'AGENT DE COLORATION DESTINÉES À ÊTRE UTILISÉES EN MICROSCOPIE À FLUORESCENCE

(30) Priority: 08.03.2019 US 201962815785 P
(43) Date of publication of application: 12.01.2022
(60) Divisional application: 25175764.7
(73) Proprietor: Samantree Medical SA, 1007 Lausanne (CH)
(72) Inventor: SHAFFER, Etienne, 1007 Lausanne (CH); HORISBERGER, Aurèle Timothée, 1022 Chavannes-Près-Renens (CH); SCHMITT, Frédéric, 1007 Lausanne (CH); RACHET, Bastien, 1007 Lausanne (CH)
(74) Representative: Graham Watt & Co LLP
(86) International application number: PCT/EP2020/055941
(87) International publication number: WO 2020/182633

(56) References cited:
- WO-A1-2014/186544
- CN-A- 109 078 167
- US-A- 5 334 509
- US-A1- 2003 027 777
- US-B1- 6 790 636
- DOBBS JESSICA L ET AL: "Feasibility of confocal fluorescence microscopy for real-time evaluation of neoplasia in fresh human breast tissue", JOURNAL OF BIOMEDICAL OPTICS, SPIE, vol. 18, no. 10, 1 October 2013 (2013-10-01), pages 106016, XP060023942, ISSN: 1083-3668, [retrieved on 20131028], DOI: 10.1117/1.JBO.18.10.106016

## Description

### TECHNICAL FIELD

This disclosure relates generally to staining agent solutions useful in fluorescence microscopy.

### BACKGROUND

Many modalities of microscopy use fluorescence to generate images. One example of a microscopy modality that typically uses fluorescence is confocal microscopy. Many samples that are desirable to image do not naturally fluoresce. For example, most biological samples are not naturally fluorescent. In order to utilize fluorescence for imaging such biological samples, the samples need to be stained with a staining agent solution. The staining agent solution tags the sample with a fluorophore. Some staining agent solutions selectively stain certain cell or tissue types or certain portions of a cell, such as DNA. Acridine orange is an example of a fluorophore commonly used in staining agent solutions to fluorescently stain biological samples for fluorescence microscopy.
Dobbs et al. (J Biomed Opt. 2013. 18:106016) use proflavine to stain cancer tissue samples.

### SUMMARY

It is presently found that commercially available staining agent solutions that include acridine orange are ill-suited for certain applications. Examples of such commercially available staining agent solutions include Remel ref. 40010 and Becton Dickinson (BD) ref. 212536. These staining agent solutions were developed specifically to stain microorganisms differentially from human cellular material. Moreover, typical staining agent solutions have several undesirable characteristics, such as noticeable smell, low pH, strong color, and toxic ingredients. There is a need for new staining agent solutions, particularly when maintaining tissue integrity is important and/or in applications where a sample will be exposed to a user during imaging, where characteristics, such as smell, color, low pH, and toxicity, can be particularly undesirable.

The present invention relates in a first aspect to a staining agent saline solution for use in intraoperative fluorescence microscopy of freshly resected tissue samples, the staining agent solution comprising acridine orange at a concentration from 0.18 g/L to 0.22 g/L, wherein the staining agent solution has a pH from 6.8 to 7.4.

The present disclosure includes staining agent solutions for use in fluorescence microscopy, such as confocal fluorescence microscopy. In certain embodiments, the staining agent solutions mitigate or eliminate one or more undesirable characteristics such as noticeable smell, low pH, strong color, and toxic ingredients.

One example of an application of fluorescence microscopy that can utilize staining agent solutions disclosed herein is intraoperative confocal microscopy. Intraoperative confocal microscopy can be used to rapidly assess resected tissue samples. For example, a common procedure is to resect cancerous tissue from a patient. Once resected, it is important to check the resected tissue mass to determine that a sufficient margin of healthy tissue surrounds the removed cancerous tissue. If the resected tissue does not have an appropriate margin, reoperation may be required adding expense and risk to the patient. To perform an assessment of tissue margins, a resected tissue sample can be stained with a fluorescent staining agent solution and confocal microscopy can be used to image a surface layer of the tissue sample. It is important in such an application that the tissue is minimally disrupted in order to produce accurate images for assessment by the surgeon performing the resection and/or allow further postoperative assessment as part of the clinical workflow. Moreover, it is desirable for the appearance and smell to be undisrupted by the staining process in order to facilitate wide adoption of the process by surgical staff and not interfere with any qualitative assessment made of the tissue sample by visual inspection.

In one aspect, the present disclosure is directed to a staining agent solution for use in fluorescence microscopy, the staining agent solution comprising acridine orange, wherein the staining agent solution has a pH from 6 to 8 (e.g., from 6.5 to 7.5).

According to the invention, the concentration of the acridine orange is from 0.18 g/L to 0.22 g/L.

According to the invention, the staining agent solution is a saline solution. In certain embodiments, the solution comprises a buffer. In certain embodiments, the buffer is a phosphate-buffered saline (PBS). In certain embodiments, the solution comprises a phosphate-buffered saline (PBS).

In certain embodiments, the acridine orange is acridine orange CAS 65-61-2. In certain embodiments, the acridine orange is acridine orange CAS 10127-02-3.

In certain embodiments, the solution is isotonic. According to the invention, the solution comprises sodium chloride. In certain embodiments, the solution has a sodium chloride concentration of from 6 g/L (e.g., 0.1 molar (M)) to 9 g/L (e.g., 1.5 M) (e.g., from 6.75 g/L to 7.25 g/L). In certain embodiments, the solution comprises potassium dihydrogen phosphate. In certain embodiments, the solution has a potassium dihydrogen phosphate concentration of less than 1 g/L (e.g., 7.3 millimolar (mM)). In certain embodiments, the solution has a potassium dihydrogen phosphate concentration of from 0.2 g/L (e.g., 1.5 mM) to 0.6 g/L (e.g., 4.4 mM) (e.g., from 0.35 g/L to 0.45 g/L). In certain embodiments, the solution comprising disodium hydrogen phosphate. In certain embodiments, the solution having a disodium hydrogen phosphate concentration of from 1 g/L (e.g., 7 mM) to 3 g/L (e.g., 21.1 mM) (e.g., from 1.25 g/L to 1.75 g/L).

In certain embodiments, the solution does not comprise acetic acid (e.g., an acetate buffer).

In a further aspect, the invention relates to a method of sample imaging, the method comprising:staining a freshly resected tissue sample with a fluorescent staining agent saline solution in an operating room or in a room adjacent to an operating room, wherein the staining agent solution comprises acridine orange in a concentration from 0.18 g/L to 0.22 g/L and has a pH from 6.8 to 7.4, wherein the tissue sample is exposed to the fluorescent staining agent solution for a period of no more than 1 minute, preferably wherein the period is from 15 seconds to 30 seconds, more preferably from 1 second to 15 seconds;disposing the tissue sample on or over a transparent imaging window of an imaging system such that the tissue sample is exposed to a user during imaging and imaging a surface of the tissue sample with the imaging system, wherein the method is performed intraoperatively.

In the method of the invention, the tissue sample is exposed to the fluorescent staining agent solution for a period of no more than 1 minute (e.g., wherein the period is from 15 seconds to 30 seconds in length or from 1 second to 15 seconds). In certain embodiments, the imaging step comprises imaging the surface of the tissue sample in no more than 5 minutes (e.g., no more than 2 minutes or no more than 90 seconds).

In certain embodiments, the method comprises reorienting the tissue sample; and imaging a second surface of the tissue sample with the imaging system.

The method is performed intraoperatively.

In certain embodiments, the imaging system is located in an operating room (e.g., in proximity to an operating table) [e.g., or in a room adjacent to an operating room (e.g., a sterilizable auxiliary room)] during the imaging. According to the method of the invention, the staining of the tissue sample occurs in an operating room (e.g., in proximity to an operating table) or in a room adjacent to an operating room (e.g., a sterilizable auxiliary room).

According to the method of the invention, the tissue sample is disposed on an exposed optical interface of a sample dish that is disposed between the tissue sample and the transparent imaging window.

According to the invention, the tissue sample is a freshly resected tissue sample (e.g., a resected tissue sample comprising cancer).

According to the invention, the fluorophore is acridine orange and wherein the staining agent solution has an acridine orange concentration from 0.18 g/L to 0.22 g/L.

### BRIEF DESCRIPTION OF THE DRAWINGS

Drawings are presented herein for illustration purposes, not for limitation. The foregoing and other objects, aspects, features, and advantages of the disclosure will become more apparent and may be better understood by referring to the following description taken in conjunction with the accompanying drawings, in which:
FIGS. 1A 1B, 1C and 1D show confocal images of different stained tissue samples resulting from comparative experimental tests;
FIG. 2A shows a fluorescently stained biological sample disposed on an imaging system, according to illustrative embodiments of the disclosure;
FIG. 2B shows a full view of the imaging system of FIG. 2A, according to illustrative embodiments of the disclosure; and
FIG. 3 is a flow diagram of a method for intraoperative imaging, according to illustrative embodiments of the disclosure.

### DETAILED DESCRIPTION OF CERTAIN EMBODIMENTS

In this application, unless otherwise clear from context or otherwise explicitly stated, (i) the term "a" may be understood to mean "at least one"; (ii) the term "or" may be understood to mean "and/or"; (iii) the terms "comprising" and "including" may be understood to encompass itemized components or steps whether presented by themselves or together with one or more additional components or steps; (iv) the terms "about" and "approximately" may be understood to permit standard variation as would be understood by those of ordinary skill in the relevant art; and (v) where ranges are provided, endpoints are included. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The invention relates to a staining agent saline solution for use in intraoperative fluorescence microscopy of freshly resected tissue samples, the staining agent solution comprising acridine orange at a concentration from 0.18 g/L to 0.22 g/L, wherein the staining agent solution has a pH from 6.8 to 7.4. A staining agent solution may differentially stain different portions of a tissue sample. For example, a staining agent solution may stain cells different from non-cellular material or may selectively stain certain portions of a cell (e.g., DNA).

### Staining agent solutions

In some embodiments, a staining agent solution comprises acridine orange (Chemical Abstracts Service registry number (CAS) 65-61-2). Acridine orange (CAS 65-61-2) has a chloride counterion. In some embodiments, a staining agent solution comprises acridine orange (CAS 10127-02-3). Acridine orange (CAS 10127-02-3) has a ZnCl₂ counterion. In some embodiments, a staining agent solution comprises both acridine orange (CAS 65-71-2) and acridine orange (CAS 10127-02-3). As used herein, "acridine orange" (without an explicitly stated CAS) is understood to refer to, in some embodiments, either of acridine orange (CAS 65-61-2) or acridine orange (CAS 10127-02-3) and, in some embodiments, a mixture of both acridine orange (CAS 65-61-2) and acridine orange (CAS 10127-02-3). Acridine orange may fluoresce at about 525 nm and may have an excitation maximum at about 500 nm (e.g., when bound to DNA). Acridine orange may fluoresce at about 650 nm and may have an excitation maximum at about 460 nm (e.g., when bound to RNA).

Proflavine (acridine-3,6-diamine) is a fluorophore that can be used to stain a sample (not part of the invention). As described herein, a staining agent solution comprises proflavine with an hemisulfate counterion (CAS 1811-28-5). Proflavine in water [with pH of from 6 to 8 (e.g., from 6.5 to 7.5) (e.g., about 7)] may fluoresce at about 515 nm, and may have a peak absorption at about 460 nm. As described herein, a staining agent solution comprises both proflavine and acridine orange. As described herein, proflavine and acridine orange are used together in order to provide a staining agent solution capable of selectively staining portions (e.g., different portions) of a sample.

Acriflavine (3,6-diamino-10-methylacridin-10-ium chloride) is a fluorophore that can be used to stain a sample (not part of the invention). Acriflavine in water [with pH of from 6 to 8 (e.g., from 6.5 to 7.5) (e.g., about 7)] may fluoresce at about 515 nm, and may have an excitation maximum at about 415 nm. As described herein, a staining agent solution comprises acriflavine. For example, a staining agent solution may comprise acriflavine (CAS 8048-52-0), which has a chloride counterion. As described herein, a fluorophore in a staining agent solution is acriflavine. As described herein, a staining agent solution comprises both acriflavine and acridine orange.

As described herein, acriflavine and acridine orange are used together in order to provide a staining agent solution capable of selectively staining portions (e.g., different portions) of a sample.

Fluorophore concentration in a staining agent solution must be sufficiently high to stain a sample, or portion thereof, but high fluorophore concentration can introduce toxicity that may degrade a tissue sample as well as may exceed the solubility limit of the fluorophore in the solution. For example, in the case of acridine orange, commercially available staining agent solutions typically have an acridine orange concentration of 0.1 g/L in the solutions. According to the invention, the staining agent saline solution comprises acridine orange at a concentration from 0.18 g/L to 0.22 g/L.

Commercially available acridine orange solutions are fairly acidic, commonly having a pH of below 6, for example around 3.5-4. Such a low pH results from use of acetic acid in an acetate-buffer solution, with its natural pH of about 4.75, and, in some case, addition of hydrochloric acid (HCl) to further lower the pH. Such a pH may be acceptable for certain applications, such as determining whether one or more microorganisms are present in a sample, but are undesirable when maintaining tissue integrity is of high importance. For example, it is desirable to maintain the integrity of tissue resected during surgery during staining both for any fluorescence imaging that is performed intraoperatively (e.g., without fixing the sample) and for any postoperative histological assessment (such as a paraffin section analysis). According to the invention, the staining agent solution has a pH from 6.8 to 7.4. In this way, the staining agent solution has a pH more closely resembling a physiological pH, thereby reducing the likelihood of adversely impacting (e.g., damaging) a tissue sample when stained. In some embodiments, a staining agent solution does not comprise acetic acid (e.g., an acetate buffer).

Solubility of a fluorophore at neutral pH can be achieved by using an appropriate solvent. In some embodiments, the solution comprises a buffer. A buffer can stabilize pH, thereby improving long-term stability of the staining agent solution. In some embodiments, the buffer is a phosphate-buffered saline (PBS). In some embodiments, the buffer is isotonic. According to the invention, the solution comprises saline. In some embodiments, the solution comprises a phosphate-buffered saline (PBS).

A solution in which a fluorophore is distributed may comprise water. Water alone may be insufficient to mitigate adverse impacts to a tissue sample. Osmotic shock may occur, thereby impacting a tissue sample (e.g., damaging fat cells), if water alone is used as the solvent for a fluorophore. Therefore, in some embodiments, the solution in which a fluorophore is distributed is isotonic. A PBS may be used in the solution to provide approximate isotonicity. Therefore, a PBS can serve a dual function of providing a physiologically compatible pH and mitigating damage caused by osmolarity or ion concentration differences.

One or more salts may be included in a solution, for example to provide desired counterions and/or tonicity. For example, a solution may include one or more of sodium chloride, potassium phosphate, and sodium phosphate. According to the invention, acridine orange is in a solution comprising sodium chloride. In some embodiments, the sodium chloride concentration is from 6 g/L to 9 g/L. In some embodiments, a fluorophore is in a solution comprising potassium dihydrogen phosphate. In some embodiments, the potassium dihydrogen phosphate concentration is less than 1 g/L (e.g., from 0.25 g/L to 0.5 g/L). In some embodiments, a fluorophore is in a solution comprising disodium hydrogen phosphate. In some embodiments, the disodium hydrogen phosphate concentration is from 1 g/L to 3 g/L.

The commonly used acetate buffer solution has another undesirable characteristic for a staining agent solution: smell. Acetic acid gives off a relatively strong and unpleasant smell. While the smell may be mild enough to tolerate in a laboratory setting, the smell can be off-putting and even potentially dangerous in an operating room or patient examination room setting. For example, a strong chemical smell may cause a surgeon to be less likely to use a staining agent solution or even to forgo intraoperative fluorescence imaging. Moreover, a strong chemical smell can mask or obfuscate other, potentially more subtle odors, which may endanger a patient being treated near where a staining agent solution is being used. For example, in an operating room, a strong chemical odor may mask or obfuscate a smell emanating from a patient indicative of an adverse change in the patient. For example, a new smell from a patient may indicate that a fluid barrier has been breached during surgery and may be masked or obfuscated by a staining agent solution with a strong smell. Therefore, reducing or eliminating strong smells from a staining agent solution not only makes the staining agent solution more pleasant to work with, but can actually improve health outcomes for patients (e.g., by leading to faster detection of a problem during surgery). A solution without acetic acid or other chemicals having strong odors is therefore preferable. A PBS can be used instead of typically-used buffer (e.g., acetate buffer) to reduce or eliminate strong smell.

By mimicking physiological conditions, such as osmolarity, ion concentration, pH or a combination thereof for example, and/or reducing toxicity, the visual appearance of a tissue sample may be better maintained during staining. Toxicity of a staining agent solution may cause cellular degradation or disruption during staining that changes the appearance of a tissue sample. Non-physiological conditions of a staining agent solution may also cause cellular degradation or disruption during staining that changes the appearance of a tissue sample. The visual appearance of a tissue sample can be important for a number of reasons, including for example qualitative assessment of tissue quality and/or composition. For example, during intraoperative tissue resection, a surgeon may qualitatively assess the tissue that has been resected to make a preliminary determination of margins and/or health status of the patient. Staining agent solutions disclosed herein more closely mimic physiological conditions and/or have reduced toxicity, as compared to commercially available staining agent solutions, such that the initial visual appearance of biological samples (e.g., freshly resected tissue) is better maintained during staining.

### Examples of staining agent solutions

A staining agent solution described herein may include acridine orange dissolved in a PBS solution at a concentration of from 0.1 g/L to 0.3 g/L, where the solution included from 6 to 8 g/L of sodium chloride, from 0.3 to 0.5 g/L of potassium dihydrogen phosphate, and from 1 to 2 g/L of disodium hydrogen phosphate. In one embodiment, a staining agent solution having the following characteristics was made and tested: 0.2 g/L of acridine orange (CAS 65-61-2) in a PBS comprising 7 g/L of sodium chloride, 0.4 g/L potassium dihydrogen phosphate, and 1.5 g/L of disodium hydrogen phosphate. The resulting solution had a pH of about 7.2, was isotonic, and did not exhibit a noticeable chemical smell. The solution was stable over a relevant time period (e.g., at least an hour). The fluorescence stability, as determined by intensity over 30 minutes after staining, was comparable or exceeded a control solution of Remel ref. 40010. In some embodiments, for example, a staining agent solution comprises 0.2 g/L of acridine orange (CAS 65-61-2) in a PBS comprising 7 g/L of sodium chloride, 0.4 g/L potassium dihydrogen phosphate, and 1.5 g/L of disodium hydrogen phosphate. Staining agent solutions described herein (not part of the invention) comprising acridine orange at a concentration of 0.4 g/L and 0.8 g/L and having a pH from 6 to 8 have also been produced, tested, and found to sufficiently stain samples to produce useable images.

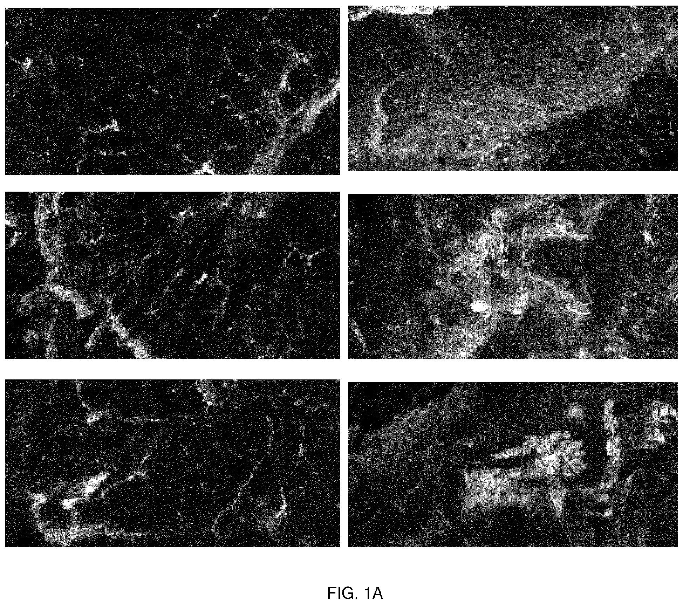

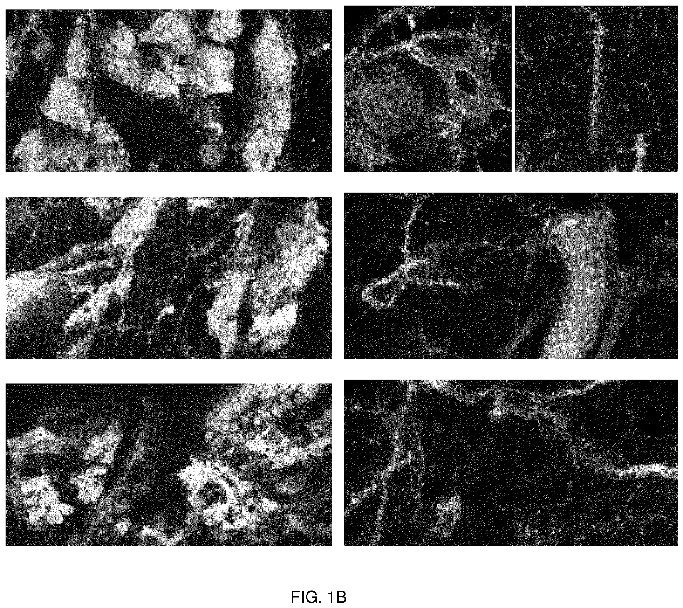

In a first comparative example (not part of the invention), a staining agent solution that includes 0.16 g/L acridine orange and 9 g/L NaCl in water was made. In a second comparative example (not part of the invention), a staining agent solution that includes 0.16 g/L acridine orange in a PBS comprising 7 g/L NaCl and 0.4 g/L potassium dihydrogen phosphate, and 1.5 g/L of disodium hydrogen phosphate was made. The staining agent solutions of the first comparative example and the second comparative example were tested and compared to a reference staining agent solution of Remel ref. 40010. In a representative comparative test, images were acquired from samples stained with the reference staining agent solution, a staining agent solution according to the first example, and a staining agent solution according to the second example. Staining of a pork breast tissue sample (approximately 8 x 8 x 20 mm in size) was performed for 30 seconds, samples were simultaneously rinsed in a common NaCl solution (e.g. 9 g/L) for approximately 6 seconds, and imaged within 30 minutes of staining. FIGS. 1A and 1B show confocal images of the stained samples from the comparative test. In FIG. 1A, the images in the top row show stained fat cells and the images in the bottom row show stained connective tissue. The left image in the bottom row of FIG. 1A also shows stained glands in addition to the stained connective tissue. In FIG. 1B, the images in the top row show stained glands and the images in the bottom row show stained vessels. The left images of the top and bottom rows of both FIG. 1A and FIG. 1B correspond to the reference solution. The center images of the top and bottom rows of both FIG. 1A and FIG. 1B correspond to the solution according to the first comparative example. The right images of the top and bottom rows of both FIG. 1A and FIG. 1B correspond to the solution according to the second comparative example. Fluorescence intensity for the sample stained with the reference solution decreased about 7% over the testing period. Fluorescence intensity for the sample stained with the solution according to the first example decayed only about 2% over the testing period. Fluorescence intensity for the sample stained with the solution according to the second example decayed only about 4% over the testing period.

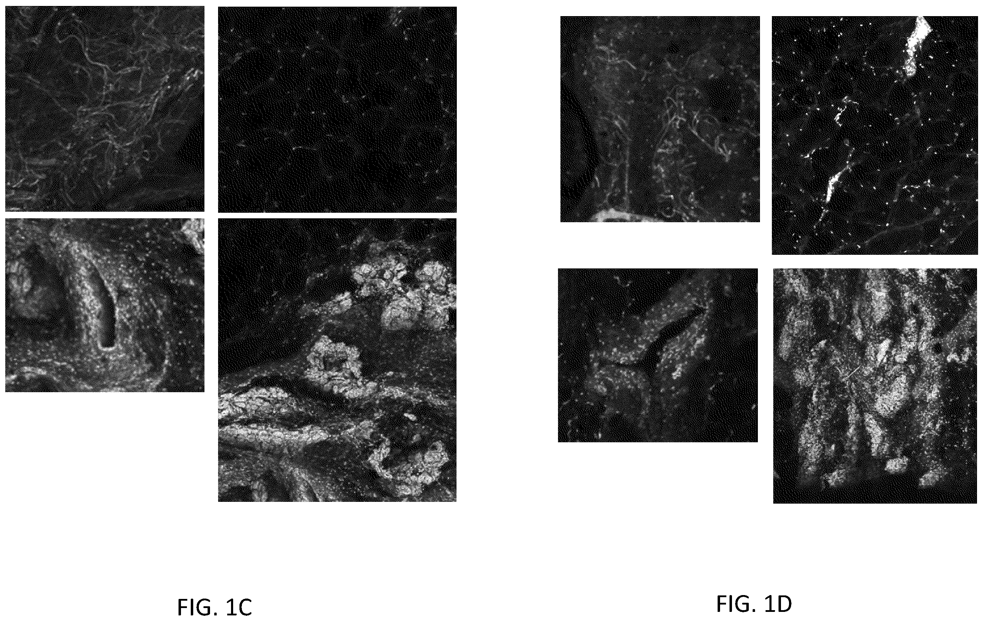

In a third example, a staining agent solution that includes 0.2 g/L acridine orange (CAS 10127-02-3) in PBS comprising 7 g/L NaCl, 0.4 g/L potassium dihydrogen phosphate and 1.5 g/L of disodium hydrogen phosphate was made. The staining agent solutions of the third example were tested and compared to a reference staining agent solution of Remel ref. 40010. In a representative comparative test, images were acquired from samples stained with the reference staining agent solution and a staining agent solution according to the third example. Staining of a pork breast tissue sample (approximately 8 x 8 x 20 mm in size) was performed for 30 seconds, samples were simultaneously rinsed in a NaCl solution for approximately 6 seconds, and imaged within 30 minutes of staining. FIG. 1C and FIG. 1D show confocal images of the stained samples from the comparative test. FIG. 1C shows images of tissue specimen stained with a reference staining agent solution of Remel ref. 40010. FIG. 1D shows images of tissue specimen stained with a staining agent solution according to the third example. In FIG. 1C and FIG. 1D, the upper left image shows stained connective tissue, the lower left image shows stained vessels, the upper right image shows stained fatty tissue and the lower right image shows stained glandular tissue. The staining agent solution of the third example provided correct and repeatable visualization of relevant morphological structures at least as well as the reference solution (by qualitative assessment). Furthermore, the staining agent solution of the third example provides reasonably stable signal over time (having ≲20% variation in fluorescence signal over 20 minutes). In some embodiments, a staining agent solution corresponding to the third example is preferred for having desirable characteristics of stability, low smell, and biocompatibility (e.g., reduced risk of osmotic shock occurring).

### Methods of use of staining agent solutions

Staining agent solutions disclosed herein are useful, for example, for fluorescence microscopy where the sample is exposed to a user (e.g., nurse, doctor, or surgeon) during imaging with an imaging system. Examples of imaging systems useful in combination with a staining agent solution disclosed herein include those described in International Patent Application Nos. PCT/EP18/79894, filed October 31, 2018, and PCT/EP18/79885, filed October 31, 2018.

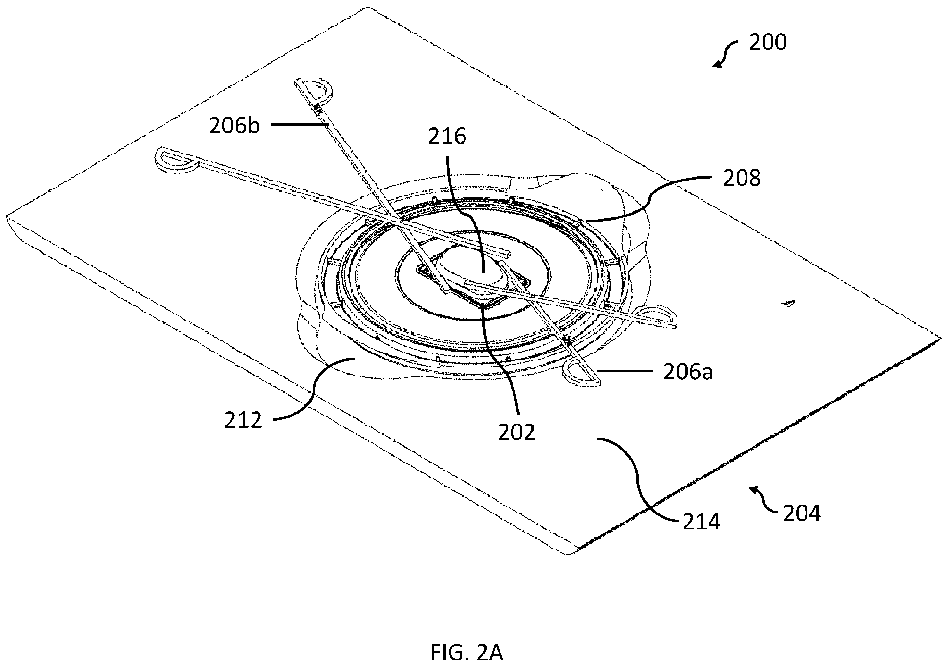

FIG. 2A shows a close up partial view of an example of an imaging system **200** with sample dish **208** disposed thereon. Tissue sample **216** (e.g., a freshly resected tissue sample) is disposed on sample dish **208.** Illustrative imaging system **200** has an exposed working area (e.g., working surface and sample) that is available to a user during imaging (i.e., the exposed working area is not covered). Tissue sample **216** can be disposed on sample dish **208** without the need for an additional sample holder. Sample dish **208** comprises an optical interface that is exposed to a user of imaging system **200** during imaging such that the user can use forceps **206a-b** (or his or her hand) to position and/or orient tissue sample **216.** Moreover, forceps **206a-b** can remain in a desired position while imaging the sample due to the exposed interface. Transparent imaging window **202** can be seen through the optical interface of sample dish **208.** Sample dish **208** is disposed between tissue sample **216** and transparent imaging window **202.** Transparent imaging window **202** allows an illumination beam and back-emitted light to pass therethrough in order to generate a fluorescence image (e.g., is at least 50% transparent to one or more wavelengths of an illumination beam and back-emitted light from one or more fluorophores in a staining agent solution).

Housing **204** comprises an imaging window support base **212** (attached to an imaging window support that holds transparent imaging window **202)** and upper working surface **214.** Imaging window support base **212** is recessed from upper working sample **214** such that when sample dish **208** is mounted onto imaging system **200,** the sample dish is positioned at or slightly below upper working surface **214.** Such an arrangement allows easy lateral access to a sample from all sides of a sample, even during imaging. Moreover, since the imaging window support base **212** is recessed from upper working surface **214** and the exposed surface of sample dish **108** is near (e.g., at or slightly below) the same plane as upper working surface **214,** tools (e.g., forceps) can easily be used and left in place during imaging (e.g., to hold a sample in a particular position). Housing **204** and transparent imaging window **202** act to isolate optics of imaging system **200** (not shown) from a tissue sample (e.g., to prevent contamination and/or damage of the optics).

A user can visually monitor and interact with tissue sample **216** during imaging with imaging system **200** because it is exposed. Moreover, imaging can be performed quite rapidly because tissue sample **216** can be easily reoriented between images. For example, a first surface of tissue sample **216** may face transparent imaging window **202** initially, such that a first image obtained by imaging system **200** corresponds to the first surface. Then, tissue sample **216** can be quickly reoriented (without needing to temporarily disassemble or open a sample holder) to image a second surface of tissue sample **216.** For example, a tissue sample may be rotated by some number of degrees (e.g., 90 degrees) or flipped over. In some examples, multiple (e.g., several or many) different region of interests of a tissue sample may be imaged (e.g., with different orientations, such as regions on opposite sides of the tissue sample) and the tissue sample thus needs to be repositioned at least once (e.g., several or many times) in order to image all of the regions of interest.

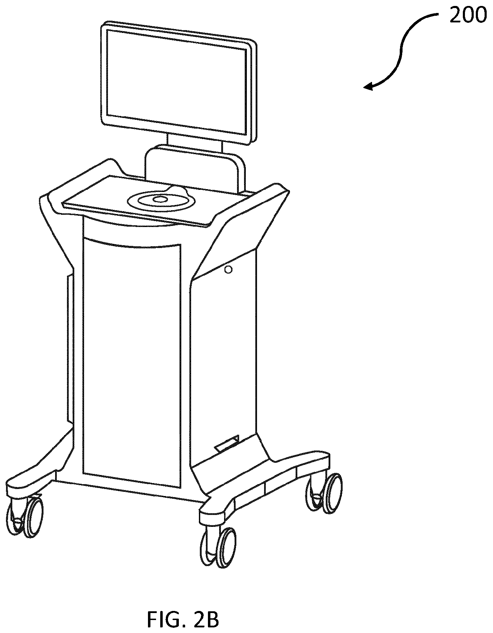

FIG. 2B shows a full view of imaging system **200.** Imaging system **200** has lockable wheels that allow it to be easily repositioned. Imaging system **200** can be located in an operating room and can be used intraoperatively to perform assessments of freshly resected tissue.

Staining agent solutions disclosed herein may quickly sufficiently stain a sample [e.g., a surface layer of the sample to a certain penetration depth (e.g., in a range from 0.1 mm to 1 mm, for example from 0.4 mm to 0.6 mm)] to allow fluorescence microscopy (e.g., confocal microscopy) to be performed. According to the invention, the staining agent saline solution stains a sample in a period of time that is no more than 1 minute (e.g., 15 seconds to 30 seconds or 1 second to 15 seconds). In some embodiments, samples to be imaged are relatively large (e.g., having dimensions up to 10 cm x 10 cm x 10 cm). For example, typical breast cancer tissue samples (lumps) can be as large as 8 cm in at least one dimension and thus have an area to image (full surface) up to 400 cm² (e.g., approximately equivalent to an 8 cm x 8 cm x 8 cm volume). In some embodiments, a tissue sample has a volume of from 0.25 cm³ to 500 cm³. In some embodiments, an imaging system can image up to 18 cm² (e.g., up to 10 cm², up to 12 cm², or up to 15 cm²) in a period of time of no more than 3 minutes (e.g., no more than 2 minutes, no more than 90 seconds, or no more than one minute) for example by using a large array of micro optical elements to perform a large area parallel scan. Staining agent solutions disclosed herein are capable of sufficiently staining a tissue sample to produce high quality fluorescence images within the aforementioned short time period. In some embodiments, no more than 50 images (e.g., no more than 40 images or no more than 30 images) and thus less than 60 minutes (e.g., less than 40 minutes or less than 30 minutes) are needed to image the entire surface of a large sample. Less time (and less images) is (are) needed to image smaller samples. In certain embodiments, a full sample surface (e.g., of a resected tissue sample comprising cancer) is imaged during an intraoperative assessment.

According to the invention, staining and/or imaging of a stained tissue sample with an imaging system occurs intraoperatively, thereby allowing assessments of images to be made during the surgery. That is, staining and/or imaging of a stained tissue sample with an imaging system occurs in an operating room or a room adjacent to an operating room (e.g., a sterilizable auxiliary room). For example, an imaging system may be located in an operating room (e.g., in proximity to an operating table). By locating an imaging system in an operating room and also staining samples in the operating room, time between fresh resection of tissue and image acquisition can be reduced. Adoption of such a procedure may be aided by a staining agent solution that lacks undesirable characteristics, for example strong smell.

A tissue sample may be stained, for example, by immersing or dipping the tissue sample in a beaker filled with a staining agent solution or by coating the tissue sample with the staining agent solution. In some embodiments, a tissue sample is rinsed (e.g., with or in a salt solution) after staining and prior to imaging.

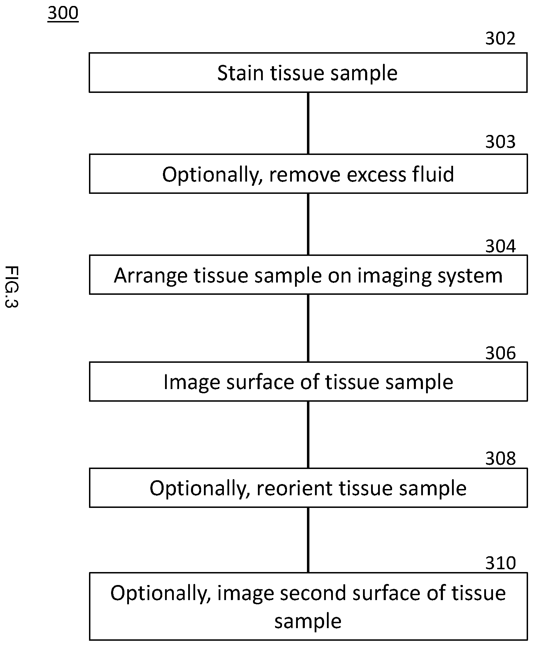

FIG. 3 is a flow diagram illustrating an exemplary method **300** of imaging a tissue sample using a staining agent solution disclosed herein (not part of the invention). In step **302,** the tissue sample is stained. The staining agent solution used to stain the tissue sample may be, for example, a staining agent solution comprising acridine orange at a concentration of from 0.1 g/L to 0.3 g/L in a solution having a pH of from 6 to 8. The tissue sample may be stained, for example, by immersing or dipping the tissue sample in a beaker filled with a staining agent solution or by coating the tissue sample with the staining agent solution. The staining agent solution may be applied to the tissue sample for a period of time of no more than 1 minute (e.g., a period of from 15 seconds to 30 seconds). In step **303,** excess fluid (e.g., stain or blood) may be optionally removed after staining, for example by rinsing with saline solution and/or by using a wipe or towel. In step **304,** the tissue sample is arranged on an imaging system for imaging. The imaging system may be, for example, illustrative imaging system **200** discussed with reference to FIGS. 2A and 2B. In some aspects, the tissue sample is exposed (e.g., accessible) to a user after being arranged for imaging (e.g., and throughout imaging). In step **306,** a surface of the tissue sample is imaged (e.g., in no more than three minutes). In optional step **308,** the tissue sample is reoriented to image a second surface of the sample (e.g., in order to perform a full surface mapping). In optional step **310,** the second surface of the sample is imaged. Images obtained during the method can be of higher quality because the staining agent solution does not disrupt or degrade the tissue sample.

## Claims

1. A staining agent saline solution for use in intraoperative fluorescence microscopy of freshly resected tissue samples, the staining agent solution comprising acridine orange at a concentration from 0.18 g/L to 0.22 g/L, wherein the staining agent solution has a pH from 6.8 to 7.4.

2. The staining agent solution of claim 1, the saline solution comprising a buffer, preferably a phosphate-buffered saline (PBS).

3. The staining agent solution of any one of the preceding claims, wherein the solution is isotonic.

4. The staining agent solution of any one of the preceding claims, wherein the acridine orange is acridine orange CAS 65-61-2.

5. The staining agent solution of any one of claims 1-3, wherein the acridine orange is acridine orange CAS 10127-02-3.

6. The staining agent solution of any one of the preceding claims, the solution having a sodium chloride concentration from 6 g/L to 9 g/L.

7. The staining agent solution of any one of the preceding claims, comprising potassium dihydrogen phosphate, preferably wherein the solution has a potassium dihydrogen phosphate concentration of less than 1 g/L, preferably from 0.2 g/L to 0.6 g/L.

8. The staining agent solution of any one of the preceding claims, the solution comprising disodium hydrogen phosphate, preferably wherein the solution has a disodium hydrogen phosphate concentration of from 1 g/L to 3 g/L.

9. The staining agent solution of any one of the preceding claims, wherein the solution does not comprise acetic acid.

10. A method of sample imaging, the method comprising:
staining a freshly resected tissue sample with a fluorescent staining agent saline solution in an operating room or in a room adjacent to an operating room, wherein the staining agent solution comprises acridine orange in a concentration from 0.18 g/L to 0.22 g/L and has a pH from 6.8 to 7.4, wherein the tissue sample is exposed to the fluorescent staining agent solution for a period of no more than 1 minute, preferably wherein the period is from 15 seconds to 30 seconds, more preferably from 1 second to 15 seconds;
disposing the tissue sample on or over a transparent imaging window of an imaging system such that the tissue sample is exposed to a user during imaging and
imaging a surface of the tissue sample with the imaging system, wherein the method is performed intraoperatively.

11. The method of claim 10, wherein the imaging step comprises imaging the surface of the tissue sample in no more than 5 minutes, preferably no more than 2 minutes or no more than 90 seconds.

12. The method of any one of claims 10-11, comprising:
reorienting the tissue sample; and
imaging a second surface of the tissue sample with the imaging system.

13. The method of any one of claims 10-12, wherein the tissue sample is disposed on an exposed optical interface of a sample dish that is disposed between the tissue sample and the transparent imaging window.

14. The method of any one of claims 10-13, wherein the tissue sample is a resected tissue sample, preferably a resected tissue sample comprising cancer.

## Patentansprüche

1. Färbemittel-Salzlösung zur Verwendung in der intraoperativen Fluoreszenzmikroskopie von frisch resezierten Gewebeproben, wobei die Färbemittellösung Acridinorange in einer Konzentration von 0,18 g/l bis 0,22 g/l umfasst, wobei die Färbemittellösung einen pH-Wert von 6,8 bis 7,4 aufweist.

2. Färbemittellösung nach Anspruch 1, wobei die Salzlösung einen Puffer, vorzugsweise eine phosphatgepufferte Salzlösung (PBS), umfasst.

3. Färbemittellösung nach einem der vorhergehenden Ansprüche, wobei die Lösung isotonisch ist.

4. Färbemittellösung nach einem der vorhergehenden Ansprüche, wobei es sich bei dem Acridinorange um Acridinorange CAS 65-61-2 handelt.

5. Färbemittellösung nach einem der Ansprüche 1 bis 3, wobei es sich bei dem Acridinorange um Acridinorange CAS 10127-02-3 handelt.

6. Färbemittellösung nach einem der vorhergehenden Ansprüche, wobei die Lösung eine Natriumchloridkonzentration von 6 g/l bis 9 g/l aufweist.

7. Färbemittellösung nach einem der vorhergehenden Ansprüche, umfassend Kaliumdihydrogenphosphat, wobei die Lösung vorzugsweise eine Kaliumdihydrogenphosphatkonzentration von weniger als 1 g/l, vorzugsweise von 0,2 g/l bis 0,6 g/l aufweist.

8. Färbemittellösung nach einem der vorhergehenden Ansprüche, wobei die Lösung Dinatriumhydrogenphosphat umfasst, wobei die Lösung vorzugsweise eine Dinatriumhydrogenphosphatkonzentration von 1 g/l bis 3 g/l aufweist.

9. Färbemittellösung nach einem der vorhergehenden Ansprüche, wobei die Lösung keine Essigsäure umfasst.

10. Verfahren zur Probenabbildung, wobei das Verfahren Folgendes umfasst:
Färben einer frisch resezierten Gewebeprobe mit einer fluoreszierenden Färbelösung in Kochsalzlösung im Operationssaal oder in einem angrenzenden Raum, wobei die Färbelösung Acridinorange in einer Konzentration von 0,18 g/l bis 0,22 g/l enthält und einen pH-Wert von 6,8 bis 7,4 aufweist, wobei die Gewebeprobe der Lösung des fluoreszierenden Färbemittels für einen Zeitraum von nicht mehr als 1 Minute ausgesetzt wird, wobei der Zeitraum vorzugsweise 15 Sekunden bis 30 Sekunden, stärker bevorzugt 1 Sekunde bis 15 Sekunden beträgt;
Anordnen der Gewebeprobe auf oder über einem transparenten Abbildungsfenster eines Abbildungssystems, so dass die Gewebeprobe während der Abbildung einem Benutzer ausgesetzt ist, und
Abbilden einer Oberfläche der Gewebeprobe mit dem Abbildungssystem, wobei das Verfahren intraoperativ durchgeführt wird.

11. Verfahren nach Anspruch 10, wobei der Abbildungsschritt das Abbilden der Oberfläche der Gewebeprobe in nicht mehr als 5 Minuten, vorzugsweise nicht mehr als 2 Minuten oder nicht mehr als 90 Sekunden umfasst.

12. Verfahren nach einem der Ansprüche 10 bis 11, umfassend:
Neuausrichten der Gewebeprobe; und
Abbilden einer zweiten Oberfläche der Gewebeprobe mit dem Abbildungssystem.

13. Verfahren nach einem der Ansprüche 10 bis 12, wobei die Gewebeprobe auf einer freiliegenden optischen Schnittstelle einer Probenschale angeordnet wird, die zwischen der Gewebeprobe und dem transparenten Abbildungsfenster angeordnet ist.

14. Verfahren nach einem der Ansprüche 10 bis 13, wobei die Gewebeprobe eine resektierte Gewebeprobe ist, vorzugsweise eine resektierte Gewebeprobe, die Krebs umfasst.

## Revendications

1. Solution saline d'agent de coloration destinée à être utilisée dans la microscopie à fluorescence peropératoire d'échantillons de tissus fraîchement réséqués, la solution d'agent de coloration comprenant de l'orange d'acridine à une concentration de 0,18 g/l à 0,22 g/l, dans laquelle la solution d'agent de coloration a un pH de 6,8 à 7,4.

2. Solution d'agent de coloration selon la revendication 1, la solution saline comprenant un tampon, de préférence une solution saline tamponnée au phosphate (PBS).

3. Solution d'agent de coloration selon l'une quelconque des revendications précédentes, dans laquelle la solution est isotonique.

4. Solution d'agent de coloration selon l'une quelconque des revendications précédentes, dans laquelle l'orange d'acridine est l'orange d'acridine CAS 65-61-2.

5. Solution d'agent de coloration selon l'une quelconque des revendications 1 à 3, dans laquelle l'orange d'acridine est l'orange d'acridine CAS 10127-02-3.

6. Solution d'agent de coloration selon l'une quelconque des revendications précédentes, la solution ayant une concentration en chlorure de sodium de 6 g/l à 9 g/l.

7. Solution d'agent de coloration selon l'une quelconque des revendications précédentes, comprenant du dihydrogénophosphate de potassium, de préférence dans laquelle la solution a une concentration en dihydrogénophosphate de potassium inférieure à 1 g/l, de préférence de 0,2 g/l à 0,6 g/l.

8. Solution d'agent de coloration selon l'une quelconque des revendications précédentes, la solution comprenant du phosphate disodique hydrogéné, de préférence dans laquelle la solution a une concentration en phosphate disodique hydrogéné de 1 g/l à 3 g/l.

9. Solution d'agent de coloration selon l'une quelconque des revendications précédentes, dans laquelle la solution ne comprend pas d'acide acétique.

10. Procédé d'imagerie d'échantillon, le procédé comprenant :
la coloration d'un échantillon de tissu fraîchement réséqué avec une solution saline d'agent de coloration fluorescent dans une salle d'opération ou dans une salle adjacente à une salle d'opération, dans lequel la solution d'agent de coloration comprend de l'orange d'acridine dans une concentration de 0,18 g/l à 0,22 g/l et a un pH de 6,8 à 7,4**,** dans lequel l'échantillon de tissu est exposé à la solution d'agent de coloration fluorescent pendant une période ne dépassant pas 1 minute, de préférence dans lequel la période est de 15 secondes à 30 secondes, plus préférablement de 1 seconde à 15 secondes ;
la disposition de l'échantillon de tissu sur ou au-dessus d'une fenêtre d'imagerie transparente d'un système d'imagerie de sorte que l'échantillon de tissu est exposé à un utilisateur pendant l'imagerie et
l'imagerie d'une surface de l'échantillon de tissu avec le système d'imagerie, dans lequel le procédé est exécuté de manière peropératoire.

11. Procédé selon la revendication 10, dans lequel l'étape d'imagerie comprend l'imagerie de la surface de l'échantillon de tissu en pas plus de 5 minutes, de préférence pas plus de 2 minutes ou pas plus de 90 secondes.

12. Procédé selon l'une quelconque des revendications 10 et 11, comprenant :
la réorientation de l'échantillon de tissu ; et
l'imagerie d'une seconde surface de l'échantillon de tissu avec le système d'imagerie.

13. Procédé selon l'une quelconque des revendications 10 à 12, dans lequel l'échantillon de tissu est disposé sur une interface optique exposée d'un plateau d'échantillon qui est disposé entre l'échantillon de tissu et la fenêtre d'imagerie transparente.

14. Procédé selon l'une quelconque des revendications 10 à 13, dans lequel l'échantillon de tissu est un échantillon de tissu réséqué, de préférence un échantillon de tissu réséqué comprenant un cancer.
